# FASCICULE DE BREVET EUROPEEN

(11) **EP 1 342 458 B1**
(45) Date de publication et mention de la délivrance du brevet: **10.08.2005**
(21) Numéro de dépôt: 03290436.9
(22) Date de dépôt: 24.02.2003
(51) Int. Cl.: A61F 5/00

(54) **Dispositif de constriction gastrique à ballonnet en soufflet**
Magenband mit balgförmigem Ballon
Gastric constriction device having a balloon with bellow pleats

(30) Priorité: 06.03.2002 FR 0202851
(43) Date de publication de la demande: 10.09.2003
(73) Titulaire: Cousin Biotech, 59117 Wervicq Sud (FR)
(72) Inventeur: Creusy, Valérie, 59320 Englos (FR); Niville, Erik, 3600 Genk (BE)
(74) Mandataire: Thinat, Michel

(56) Documents cités:
- DE-A- 19 751 733
- US-A- 4 019 499
- US-A- 5 160 338
- US-A- 5 449 368

## Description

La présente invention concerne, de façon générale, les implants chirurgicaux.

Plus précisément, l'invention concerne un dispositif de constriction gastrique applicable au traitement de l'obésité morbide, ce dispositif comprenant un ballonnet creux délimitant un volume interne, des organes de maintien mâle et femelle, solidaires du ballonnet en des première et seconde zones respectives du ballonnet distantes l'une de l'autre, pour maintenir ce ballonnet dans une configuration opérationnelle dans laquelle il est refermé sur lui-même en formant un anneau entourant au moins partiellement un axe principal, et un conduit d'alimentation communiquant avec le volume interne du ballonnet.

Un dispositif selon le préambule de la revendication 1 est décrit dans le document DE 19751733.

Des dispositifs de ce type sont déjà connus et utilisés pour éviter les effets néfastes de surcharges pondérales, dans des cas sérieux.

Après mise en place d'un tel dispositif autour de l'estomac, un liquide est injecté dans le ballonnet, qui se gonfle en enserrant le tube digestif, réduisant le volume opérationnel de l'estomac, et obligeant physiquement le patient à limiter ses prises de nourriture.

Un problème fréquemment rencontré dans les dispositifs de ce type réside dans le fait que le liquide injecté dans le ballonnet subit une pression relativement élevée, de sorte qu'il a tendance à suinter hors du ballonnet, dont l'état distendu augmente la porosité.

Dans ce contexte, la présente invention a principalement pour but de proposer un dispositif de constriction gastrique exempt de ce défaut.

A cette fin, le dispositif de constriction gastrique de l'invention, par ailleurs conforme à la définition générique qu'en donne le préambule ci-dessus, est essentiellement caractérisé en ce que le ballonnet présente, en section axiale de l'anneau, une paroi annulaire radialement externe, une paroi annulaire radialement interne, et des parois axiales ondulées, disposées de part et d'autre d'un plan médian transversal à l'axe principal de l'anneau, s'étendant chacune entre les parois externe et interne du ballonnet, et conférant à ce ballonnet une structure en soufflet propre à subir, par remplissage du volume interne du ballonnet, une expansion radiale au moins en direction de l'axe principal de l'anneau.

De préférence, les parois externe et interne présentent des épaisseurs respectives différentes, l'épaisseur de la paroi externe étant supérieure à celle de la paroi interne.

Avantageusement, la paroi interne du ballonnet, en configuration opérationnelle de ce dernier, entoure totalement l'axe principal.

La paroi externe peut présenter deux surépaisseurs locales dont chacune est adjacente à l'une des parois axiales.

Le dispositif de l'invention peut alors comprendre un renfort textile disposé dans l'espace séparant les deux surépaisseurs et empêchant l'extension radiale externe du ballonnet lors de son gonflement.

Le conduit d'alimentation est par exemple solidaire d'une première extrémité du ballonnet et présente un épaulement distant de la première extrémité du ballonnet, l'organe de maintien mâle étant alors formé par une restriction du conduit s'étendant entre la première extrémité du ballonnet et l'épaulement du conduit.

L'organe de maintien femelle peut comprendre un collier de serrage ouvert, solidaire d'une seconde extrémité du ballonnet et comprenant deux joues de serrage disposées en regard l'une de l'autre, ce collier enserrant sélectivement la restriction du conduit par rapprochement et par liaison réciproque de ses joues de serrage, et offrant à la restriction du conduit, par libération réciproque des joues de serrage et écartement de ces dernières, un passage permettant l'ouverture de l'anneau.

La restriction du conduit suit de préférence une orientation sensiblement tangentielle par rapport au ballonnet, le collier de serrage, en configuration opérationnelle du ballonnet, s'étendant dans un plan sensiblement transversal à la restriction du conduit.

En configuration opérationnelle du ballonnet, la paroi externe du ballonnet à la seconde extrémité de ce dernier est alors avantageusement plus éloignée de la première extrémité du ballonnet que ne l'est la paroi interne.

Il est possible de prévoir que l'organe de maintien femelle comprenne un organe de liaison formé d'une languette de fermeture reliée à une première des joues de serrage par un lien souple et extensible, et que cet organe de liaison adopte sélectivement une configuration d'ouverture dans laquelle la languette est libre et libère les joues de serrage, et une configuration de fermeture dans laquelle le lien souple et extensible relie l'une à l'autre les joues de serrage sous allongement élastique, et dans laquelle la languette est engagée dans une cuvette de la seconde joue de serrage.

L'épaulement du conduit peut présenter une section transversale sensiblement rectangulaire.

Les joues de serrage de l'organe de maintien femelle sont par exemple disposées de part et d'autre du plan médian transversal à l'axe principal de l'anneau.

Le dispositif de constriction gastrique de l'invention peut être réalisé au moins partiellement en silicone.

D'autres caractéristiques et avantages de l'invention ressortiront clairement de la description qui en est faite ci-après, à titre indicatif et nullement limitatif, en référence aux dessins annexés, dans lesquels:
- la Figure 1 est une vue de dessus d'un dispositif conforme à l'invention, dont l'organe de liaison est représenté en configuration d'ouverture;
- la Figure 2 est une vue en coupe du ballonnet équipant le dispositif de la figure 1, la coupe étant observée suivant l'incidence définie par les flèches II-II de la figure 1;
- la Figure 3 est une vue de côté du dispositif illustré à la figure 1;
- la Figure 4 est une vue de côté du dispositif illustré à la figure 1, mais dont l'organe de liaison est représenté en configuration de fermeture;
- la Figure 5 est une vue de dessus du dispositif illustré à la figure 4; et
- la Figure 6 est une vue en coupe du dispositif illustré à la figure 5, la coupe étant observée suivant l'incidence définie par les flèches VI-VI de la figure 5.

Comme annoncé précédemment, l'invention concerne un dispositif de constriction gastrique applicable au traitement de l'obésité morbide.

Un tel dispositif comprend de façon connue un ballonnet 1, des organes de maintien mâle et femelle 3 et 4, et un conduit d'alimentation 5.

Le ballonnet 1, par exemple réalisé en silicone, est creux et délimite un volume interne V communiquant avec le conduit d'alimentation 5.

Les organes de maintien mâle et femelle 3 et 4 sont solidaires de deux extrémités respectives 11 et 12 du ballonnet 1, distantes l'une de l'autre, et ont pour fonction de maintenir ce ballonnet dans une configuration opérationnelle dans laquelle il est refermé sur lui-même en formant un anneau entourant au moins partiellement un axe principal Z.

Selon un aspect fondamental de l'invention, le ballonnet 1 présente une structure en soufflet.

Plus précisément, le ballonnet 1 présente, dans une section qui coupe l'anneau suivant son axe Z, une paroi annulaire radialement externe 21, une paroi annulaire radialement interne 22, et deux parois axiales ondulées, 23 et 24, qui sont disposées de part et d'autre du plan médian P transversal à l'axe principal Z de l'anneau.

Les parois axiales 23 et 24 s'étendent chacune entre les parois externe et interne 21 et 22 du ballonnet pour conférer à ce dernier sa structure en soufflet.

Grâce à cette structure, le ballonnet 1 peut subir une expansion radiale, en direction de l'axe principal Z de l'anneau, par remplissage de son volume interne V sous une pression très modérée.

Pour privilégier le gonflement du ballonnet 1 vers le centre de l'anneau, la paroi externe 21 présente de préférence une épaisseur E21 supérieure à l'épaisseur E22 de la paroi interne 22.

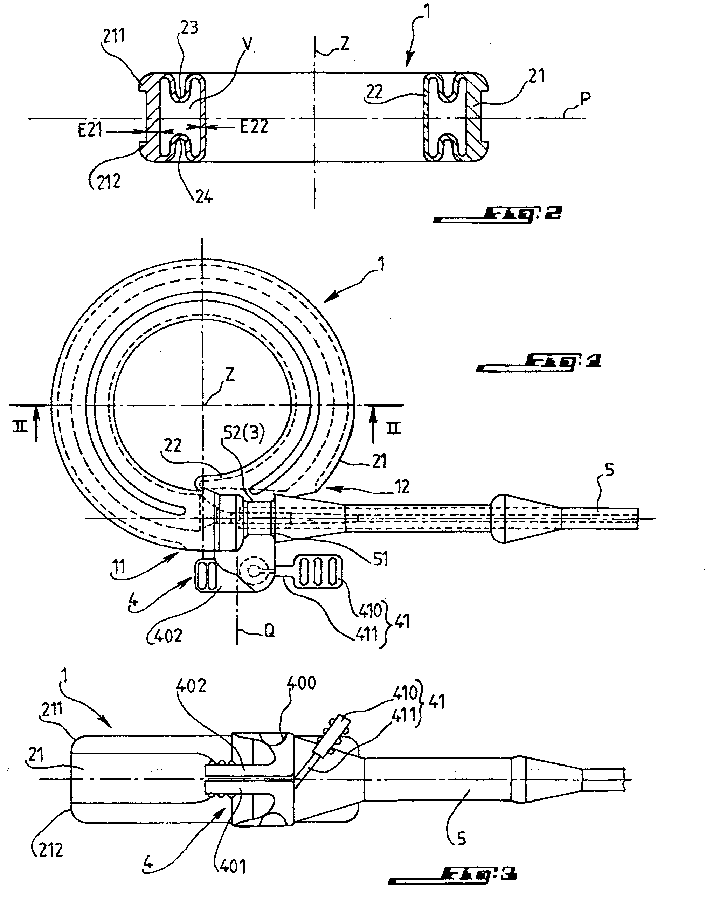

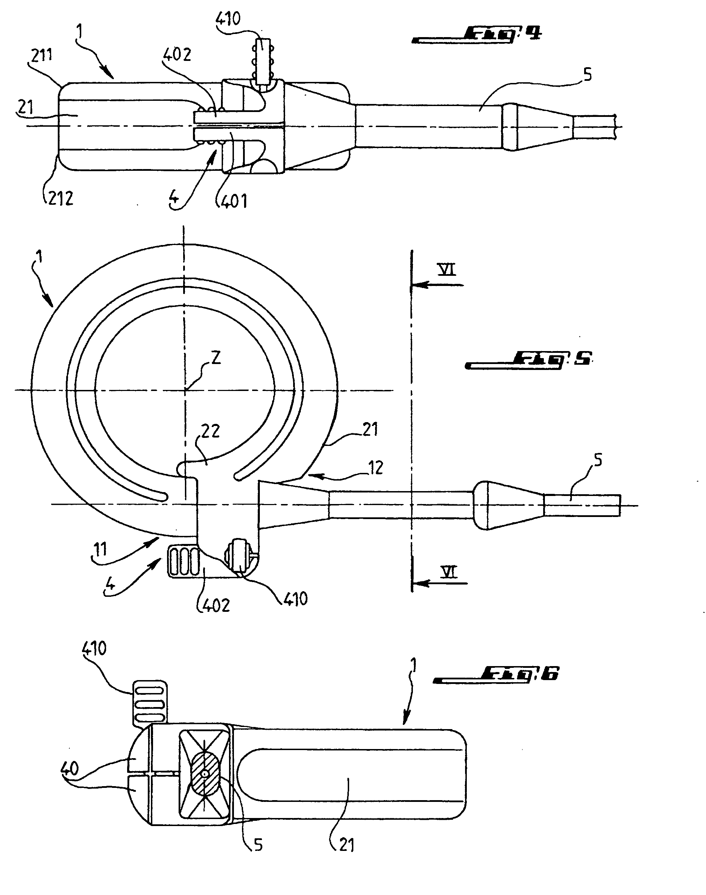

Comme le montre la figure 2, la paroi externe 21 peut présenter deux surépaisseurs locales, 211 et 212, dont chacune est adjacente à l'une des parois axiales 23 et 24 du ballonnet 1.

L'espace défini entre les deux surépaisseurs peut ainsi permettre la mise en place d'un renfort textile empêchant l'expansion externe de l'anneau lors de son gonflement.

De préférence, la paroi interne 22 du ballonnet 1, en configuration opérationnelle de ce dernier, entoure totalement l'axe principal Z, de sorte que la pression exercée par le ballonnet sur l'estomac se répartit de façon idéale.

Le conduit d'alimentation 5 est solidaire de l'extrémité 11 du ballonnet 1 et présente, à distance de cette extrémité 11, un épaulement 51 présentant par exemple une section transversale sensiblement rectangulaire (figure 6).

Le conduit d'alimentation 5 présente par ailleurs une restriction 52, qui s'étend entre l'extrémité 11 du ballonnet 1 et l'épaulement 51, et qui forme l'organe de maintien mâle 3.

L'organe de maintien femelle 4 comprend quant à lui un collier de serrage ouvert 40, solidaire de l'extrémité 12 du ballonnet 1.

Ce collier 40 comporte deux joues de serrage, 401 et 402, disposées en regard l'une de l'autre. 11 et par exemple sensiblement symétriques l'une de l'autre par rapport au plan médian P du ballonnet, qui est transversal à l'axe principal Z de l'anneau.

Lorsque les joues 401 et 402 sont rapprochées l'une de l'autre et liées entre elles, le collier 40 enserre la restriction 52 du conduit 5 et maintient ainsi le ballonnet 1 dans sa configuration en anneau.

Lorsqu'en revanche les joues 401 et 402 sont libérées et écartées l'une de l'autre, le collier 40 offre à la restriction 52 du conduit 5 un passage permettant l'ouverture de l'anneau.

Pour assurer la liaison des joues 401 et 402, l'organe de maintien femelle 4 comprend de préférence un organe de liaison 41 formé d'une languette de fermeture 410 reliée à la joue de serrage 401 par un lien souple et extensible 411.

Lorsque la languette 410 est libre, comme le montrent les figures 1 et 3, c'est-à-dire lorsque l'organe de liaison 41 est dans une configuration d'ouverture, les joues de serrage 401 et 402 peuvent être librement écartées.

L'organe de liaison peut cependant être placé dans une configuration de fermeture, dans laquelle la languette 410 est engagée dans une cuvette 400 de la seconde joue de serrage 402, et dans laquelle les joues de serrage 401 et 402 se trouvent donc reliées l'une à l'autre par le lien souple et extensible 411, placé sous léger allongement élastique pour éviter tout passage intempestif en configuration d'ouverture.

Comme le montrent le mieux les figures 1 et 5, la restriction 52 du conduit 5 suit une orientation sensiblement tangentielle par rapport au ballonnet 1, et le collier de serrage 40, en configuration opérationnelle du ballonnet 1, s'étend dans un plan Q sensiblement transversal à la restriction 52 du conduit 5.

De même, pour permettre au ballonnet 1 d'entourer totalement l'axe principal Z de l'anneau, il peut être utile de faire en sorte qu'en configuration opérationnelle du ballonnet 1, la paroi externe 21 du ballonnet, du côté de l'extrémité 12 du ballonnet 1 qui porte l'organe de maintien femelle 4, soit plus éloignée de l'autre extrémité 11 du ballonnet 1 que ne l'est la paroi interne 22 de ce dernier.

## Revendications

1. Dispositif de constriction gastrique applicable au traitement de l'obésité morbide, ce dispositif comprenant un ballonnet creux (1) délimitant un volume interne (V), des organes de maintien mâle et femelle (3, 4), solidaires du ballonnet (1) en des première et seconde zones respectives (11, 12) du ballonnet (1) distantes l'une de l'autre, pour maintenir ce ballonnet dans une configuration opérationnelle dans laquelle il est refermé sur lui-même en formant un anneau entourant au moins partiellement un axe principal (Z), et un conduit d'alimentation (5) communiquant avec le volume interne (V) du ballonnet (1), **caractérisé en ce que** le ballonnet (1) présente, en section axiale de l'anneau, une paroi annulaire radialement externe (21), une paroi annulaire radialement interne (22), et des parois axiales ondulées (23, 24), disposées de part et d'autre d'un plan médian (P) transversal à l'axe principal (Z) de l'anneau, s'étendant chacune entre les parois externe et interne (21, 22) du ballonnet, et conférant à ce ballonnet (1) une structure en soufflet propre à subir, par remplissage du volume interne (V) du ballonnet (1), une expansion radiale au moins en direction de l'axe principal (Z) de l'anneau.

2. Dispositif de constriction gastrique suivant la revendication 1, **caractérisé en ce que** les parois externe et interne (21, 22) présentent des épaisseurs respectives (E21, E22) différentes, l'épaisseur (E21) de la paroi externe (21) étant supérieure à celle (E22) de la paroi interne (22).

3. Dispositif de constriction gastrique suivant la revendication 1 ou 2, **caractérisé en ce que** la paroi interne (22) du ballonnet (1), en configuration opérationnelle de ce dernier, entoure totalement l'axe principal (Z).

4. Dispositif de constriction gastrique suivant l'une quelconque des revendications précédentes, **caractérisé en ce que** la paroi externe (21) présente deux surépaisseurs locales (211, 212) dont chacune est adjacente à l'une (23, 24) des parois axiales (23, 24).

5. Dispositif de constriction gastrique suivant la revendication 4, **caractérisé en ce qu'**il comprend un renfort textile disposé dans l'espace séparant les deux surépaisseurs (211, 212) et empêchant l'expansion radiale externe du ballonnet (1) lors de son gonflement.

6. Dispositif de constriction gastrique suivant l'une quelconque des revendications précédentes, **caractérisé en ce que** le conduit d'alimentation (5) est solidaire d'une première extrémité (11) du ballonnet (1) et présente un épaulement (51) distant de la première extrémité (11) du ballonnet (1), et **en ce que** l'organe de maintien mâle (3) est formé par une restriction (52) du conduit (5) s'étendant entre la première extrémité (11) du ballonnet (1) et l'épaulement (51) du conduit (5).

7. Dispositif de constriction gastrique suivant la revendication 6, **caractérisé en ce que** l'organe de maintien femelle (4) comprend un collier de serrage ouvert (40), solidaire d'une seconde extrémité (12) du ballonnet (1) et comprenant deux joues de serrage (401, 402) disposées en regard l'une de l'autre, ce collier (40) enserrant sélectivement la restriction (52) du conduit (5) par rapprochement et par liaison réciproque de ses joues de serrage (401, 402), et offrant à la restriction (52) du conduit (5), par libération réciproque des joues de serrage (401, 402) et écartement de ces dernières, un passage permettant l'ouverture de l'anneau.

8. Dispositif de constriction gastrique suivant la revendication 7, **caractérisé en ce que** la restriction (52) du conduit (5) suit une orientation sensiblement tangentielle par rapport au ballonnet (1), et **en ce que** le collier de serrage (40), en configuration opérationnelle du ballonnet (1), s'étend dans un plan (Q) sensiblement transversal à la restriction (52) du conduit (5).

9. Dispositif de constriction gastrique suivant la revendication 8, **caractérisé en ce qu'**en configuration opérationnelle du ballonnet (1), la paroi externe (21) du ballonnet à la seconde extrémité (12) de ce dernier est plus éloignée de la première extrémité (11) du ballonnet (1) que ne l'est la paroi interne (22).

10. Dispositif de constriction gastrique suivant l'une quelconque des revendications 7 à 9, **caractérisé en ce que** l'organe de maintien femelle (4) comprend un organe de liaison (41) formé d'une languette de fermeture (410) reliée à une première des joues de serrage (401) par un lien souple et extensible (411), et **en ce que** cet organe de liaison (41) adopte sélectivement une configuration d'ouverture dans laquelle la languette (410) est libre et libère les joues de serrage (401, 402), et une configuration de fermeture dans laquelle le lien souple et extensible (411) relie l'une à l'autre les joues de serrage (401, 402) sous allongement élastique, et dans laquelle la languette (410) est engagée dans une cuvette (400) de la seconde joue de serrage (402).

11. Dispositif de constriction gastrique suivant l'une quelconque des revendications 7 à 10, **caractérisé en ce que** les joues de serrage (401, 402) de l'organe de maintien femelle (4) sont disposées de part et d'autre du plan médian (P) transversal à l'axe principal (Z) de l'anneau.

12. Dispositif de constriction gastrique suivant l'une quelconque des revendications 6 à 11, **caractérisé en ce que** l'épaulement (51) du conduit (5) a une section transversale sensiblement rectangulaire.

## Patentansprüche

1. Magenband, anwendbar zur Behandlung krankhafter Fettleibigkeit, umfassend einen Hohlballon (1), der ein Innenvolumen (V) begrenzt, äußere und innere Halteorgane (3, 4), die in jeweiligen ersten und zweiten, voneinander beabstandeten Bereichen (11, 12) des Ballons (1) mit dem Ballon (1) einstückig sind, um diesen Ballon in einer Funktionsanordnung zu halten, in der er in sich geschlossen ist, indem er einen Ring bildet, der mindestens teilweise eine Hauptachse (Z) umgibt, sowie eine Versorgungsleitung (5), die mit dem Innenvolumen (V) des Ballons (1) in Verbindung steht, **dadurch gekennzeichnet, dass** der Ballon (1) im Axialschnitt des Rings eine radial externe ringförmige Wand (21), eine radial interne ringförmige Wand (22) und gewellte Axialwände (23, 24), die auf beiden Seiten einer mittleren Ebene (P) quer zu der Hauptachse (Z) des Ringes angeordnet sind und sich zwischen den externen und internen Wänden (21, 22) des Ballons erstrecken und diesem Ballon (1) eine balgartige Struktur verleihen, die dazu geeignet ist, durch Füllen des Innenvolumens (V) des Ballons (1) eine radiale Dehnung mindestens in Richtung der Hauptachse (Z) des Rings zu erfahren, aufweist.

2. Magenband nach Anspruch 1, **dadurch gekennzeichnet, dass** die Innen- und Außenwände (21, 22) jeweils unterschiedliche Dicken (E21, E22) aufweisen, wobei die Dicke (E21) der Außenwand (21) größer ist als die (E22) der Innenwand (22).

3. Magenband nach Anspruch 1 oder 2, **dadurch gekennzeichnet, dass** die Innenwand (22) des Ballons (1) in seiner Funktionsanordnung die Hauptachse (Z) vollständig umgibt.

4. Magenband nach einem der vorhergehenden Ansprüche, **dadurch gekennzeichnet, dass** die Außenwand (21) zwei lokale Überdicken (211, 212) aufweist, von denen jede neben einer der Axialwände (23, 24) liegt.

5. Magenband nach Anspruch 4, **dadurch gekennzeichnet, dass** es eine Stoffverstärkung umfasst, die in dem Zwischenraum angeordnet ist, der die beiden Überdicken (211, 212) trennt und beim Aufblasen die radiale externe Dehnung des Ballons (1) verhindert.

6. Magenband nach einem der vorhergehenden Ansprüche, **dadurch gekennzeichnet, dass** die Versorgungsleitung (5) mit einem ersten Ende (11) des Ballons (1) einstückig ist und einen Absatz (51) aufweist, der von dem ersten Ende (11) des Ballons (1) entfernt ist, und dass das äußere Halteorgan (3) von einer Verengung (52) der Leitung (5) gebildet wird, die sich zwischen dem ersten Ende (11) des Ballons und dem Absatz (51) der Leitung (5) erstreckt.

7. Magenband nach Anspruch 6, **dadurch gekennzeichnet, dass** das innere Halteorgan (4) eine offene Klemmschelle (40) umfasst, die mit einem zweiten Ende (12) des Ballons (1) einstückig ist und zwei Klemmbacken (401, 402) umfasst, die einander gegenüber angeordnet sind, wobei diese Schelle (40) wahlweise die Verengung (52) der Leitung (5) durch Annäherung und gegenseitige Verbindung ihrer Klemmbacken (401, 402) einklemmt und der Verengung (52) der Leitung (5) durch gegenseitige Freigabe der Klemmbacken (401, 402) und Trennen dieser Klemmbacken einen Durchgang zum Öffnen des Rings bietet.

8. Magenband nach Anspruch 7, **dadurch gekennzeichnet, dass** die Verengung (52) der Leitung (5) eine im Verhältnis zu dem Ballon (1) im Wesentlichen tangentiale Ausrichtung verfolgt, und dass die Klemmschelle (401) sich in Funktionsanordnung des Ballons (1) in einer Ebene (Q) erstreckt, die im Wesentlichen zu der Verengung (52) der Leitung (5) quer liegt.

9. Magenband nach Anspruch 8, **dadurch gekennzeichnet, dass** in Funktionsanordnung des Ballons (1) die Außenwand (21) des Ballons an dessen zweiten Ende (12) weiter von dem ersten Ende (11) des Ballons (1) entfernt ist als die Innenwand (22).

10. Magenband nach einem der Ansprüche 7 bis 9, **dadurch gekennzeichnet, dass** das innere Halteorgan (4) ein Verbindungsorgan (41) umfasst, das von einer Verschlusslasche (410) gebildet wird, die mit einer ersten der Klemmbacken (401) über ein biegsames und dehnbares Band (411) verbunden ist, und dass dieses Verbindungsorgan (41) wahlweise eine Öffnungsanordnung, in der die Lasche (410) frei ist und die Klemmbacken (401, 402) freigibt, und eine Verschlussanordnung, in der das biegsame und dehnbare Band (411) die Klemmbacken (401, 402) bei elastischer Längung miteinander verbindet, und in der die Lasche (410) in eine Senkung (400) der zweiten Klemmbacke (402) eingesteckt ist, einnimmt.

11. Magenband nach einem der Ansprüche 7 bis 10, **dadurch gekennzeichnet, dass** die Klemmbacken (401, 402) des inneren Halteorgans (4) auf beiden Seiten der mittleren Ebene (P) quer zu der Hauptachse (Z) des Ringes angeordnet sind.

12. Magenband nach einem der Ansprüche 6 bis 11, **dadurch gekennzeichnet, dass** der Absatz (51) der Leitung (5) einen im Wesentlichen rechtwinkligen Querschnitt aufweist.

## Claims

1. A gastric constriction device applicable to the treatment of morbid obesity, this device comprising a hollow balloon (1) delimiting an internal volume (V), male and female holding members (3, 4), integral with the balloon (1) in respective first and second areas (11, 12) of the balloon (1) distant from one another, for holding this balloon in operational configuration in which it is closed on itself by forming a ring encircling at least partly one main axis (Z), and a feeding conduit (5) communicating with the internal volume (V) of the balloon (1), **characterized in that** the balloon (1) has in an axial section of the ring, a radially external annular wall (21), a radially internal annular wall (22), and corrugated axial walls (23, 24), positioned on either side of a middle plane (P) transverse to the main axis (Z) of the ring, each extending between the internal and external walls (21, 22) of the balloon, and imparting to this balloon (1) a bellow structure able to undergo, by filling the internal volume (V) of the balloon (1), a radial expansion at least in the direction of the main axis (Z) of the ring.

2. The gastric constriction device according to claim 1, **characterized in that** the external and internal walls (21, 22) have different respective thicknesses (E21, E22), the thickness (E21) of the external wall (21) being larger than that (E22) of the internal wall (22).

3. The gastric constriction device according to claim 1 or 2, **characterized in that** the internal wall (22) of the balloon (1), in the operational configuration of the latter, totally encircles the main axis (Z).

4. The gastric constriction device according to any of the preceding claims, **characterized in that** the external wall (21) has two local extra thicknesses (211, 212), each of which is adjacent to one (23, 24) of the axial walls (23, 24).

5. The gastric constriction device according to claim 4, **characterized in that** it comprises a textile strengthening piece positioned in the space separating both extra thicknesses (211, 212) and preventing external radial expansion of the balloon (1) when it is inflated.

6. The gastric constriction device according to any of the preceding claims, **characterized in that** the feeding conduit (5) is integral with a first end (11) of the balloon (1) and has a shoulder (51) distant from the first end (11) of the balloon (1) and **in that** the male holding member (3) is formed by a restriction (52) of the conduit (5) extending between the first end (11) of the balloon and the shoulder (51) of the conduit (5).

7. The gastric constriction device according to claim 6, **characterized in that** the female holding member (4) comprises an open clamping collar (40), integral with a second end (12) of the balloon (1) and comprising two clamping cheeks (401, 402) positioned facing each other, this collar (40) tightly encircling the restriction (52) of the conduit (5), selectively, by bringing together and reciprocally connecting its clamping cheeks (401, 402) and providing the restriction (52) of the conduit (5), by reciprocally releasing the clamping cheeks (401, 402) and spacing apart the latter, with a passage enabling the ring to be opened.

8. The gastric constriction device according to claim 7, **characterized in that** the restriction (52) of the conduit (5) follows a substantially tangential orientation with respect to the balloon (1), and **in that** the clamping collar (40) in the operational configuration of the balloon (1) extends in a plane (Q) substantially transverse to the restriction (52) of the conduit (5).

9. The gastric constriction device according to claim 8, **characterized in that** in the operational configuration of the balloon (1), the external wall (21) of the balloon at the second end (12) of the latter, is more away from the first end (11) of the balloon (1) than is the internal wall (22).

10. The gastric constriction device according to any of claims 7 to 9, **characterized in that** the female supporting member (4) comprises a connection member (41) formed by a closing tab (410) connected to a first of the clamping cheeks (401) through a flexible and extensible link (411), and **in that** this connecting connection (41) selectively adopts an opening configuration in which the tab (410) is free and releases the clamping cheeks (401, 402) and a closing configuration in which the extensible and flexible link (411) connects the clamping cheeks (401, 402) to each other under elastic elongation, and in which the tab (410) is engaged in a cup (400) of the second clamping cheek (402).

11. The gastric constriction device according to any of claims 7 to 10, **characterized in that** the clamping cheeks (401, 402) of the female holding member (4) are positioned on either side of the middle plane (P) transverse to the main axis (Z) of the ring.

12. The gastric constriction device according to any of claims 6 to 11, **characterized in that** the shoulder (51) of the conduit (5) has a substantially rectangular transverse cross-section.
